# FASCICULE DE BREVET EUROPEEN

(11) **EP 0 522 965 B1**
(45) Date de publication et mention de la délivrance du brevet: **06.03.1996**
(21) Numéro de dépôt: 92401981.3
(22) Date de dépôt: 09.07.1992
(51) Int. Cl.: A61K 7/16

(54) **Composition à usage buccal destinée à inhiber ou détruire la mauvaise haleine et procédé d'hygiène buccale pour détruire la mauvaise haleine**
Präparat zur oralen Verwendung zur Hemmung oder Beseitigung von übelriechendem Atem sowie Verfahren zur Mundhygiene zur Beseitigung übelriechendem Atem
Oral composition to reduce or eliminate bad breath and process of oral hygiene for eliminating bad breath

(30) Priorité: 12.07.1991 FR 9108853
(43) Date de publication de la demande: 13.01.1993
(73) Titulaire: L'OREAL, F-75008 Paris (FR)
(72) Inventeur: Darmenton, Patrick, F-92320 Bourg-la-Reine (FR)
(74) Mandataire: Casalonga, Axel

(56) Documents cités:
- EP-A- 0 009 540
- US-A- 3 959 508
- US-A- 3 975 310
- US-A- 4 226 892

## Description

La présente invention concerne l'utilisation de certains composés dans des compositions à usage buccal destinées à inhiber ou à détruire la mauvaise haleine, ainsi qu'un procédé les mettant en oeuvre.

La mauvaise haleine ou haleine fétide peut avoir une cause toxique exogène comme le tabagisme et l'éthylisme, ou endogène d'ordre métabolique comme l'urémie, buccale comme la pyorrhée ou la stomatite, nasale comme la sinusite ou l'amygdalite, digestive comme la gastrite ou la colite, ou encore pulmonaire comme une dilatation des bronches ou un abcès.

Cependant, on estime globalement que dans 56 à 85% des cas, la mauvaise haleine provient de désordres dans la cavité buccale.

Le phénomène de l'haleine fétide ou halitose encore dénommée cacosmie peut constituer un réel handicap social. Environ 50% des adultes présentent une mauvaise haleine matinale.

Pour traiter cette affection, on a jusqu'ici proposé certains extraits végétaux, des sels solubles de métaux, des composés minéraux, des enzymes et certains composés organiques.

La demanderesse vient maintenant de découvrir que, de façon surprenante, une composition à usage buccal renfermant du 2-crotonyl 1,1,3-triméthyl cyclohexène-2, du 2-crotonyl 1,1,3-triméthyl cyclohexène-3, et/ou du 2-crotonyl 1,1,3-triméthyl cyclohexadiène-2,4, permettait d'inhiber ou de détruire la mauvaise haleine.

La présente invention concerne donc l'utilisation d'un composé de formule (I) : 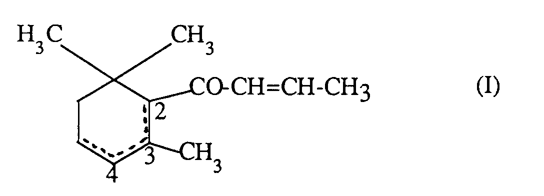
dans laquelle les pointillés représentent une double liaison en position 2 ou 3 ou deux doubles liaisons en positions 2 et 4, en vue d'inhiber ou détruire la mauvaise haleine au moyen d'une composition à usage buccal, comportant au moins l'un desdits composés et un véhicule physiologiquement acceptable.

Les composés de formule (I) sont connus.

US-A-3 959 508 décrit des compositions contenant l'un de ces composés et destinées à modifier l'arôme ou le parfum de produits alimentaires, cosmétiques ou pharmaceutiques.

Les compositions comportant le ou les composés utilisés selon l'invention sont particulièrement efficaces, comme cela a été montré selon une méthode apparentée à celle décrite par TONZETICH J., dans Périodontol, 1977, V 48, n°1, p.13-20. L'identification des composés malodorants étant réalisée par analyse chromatographique gazeuse avec détection à photo-ionisation (GC/PID) sur la phase surnageante gazeuse d'une salive incubée 2 heures à 40°C.

Préférentiellement, la composition à usage buccal renferme une quantité efficace de 2-crotonyl 1,1,3-triméthyl cyclohexadiène-2,4.

Les compositions renfermant au moins un composé de formule (I) utilisé selon l'invention peuvent se présenter sous diverses formes et notamment sous forme de sprays, de mousses, de bains de bouche, de gargarismes, de poudres, de comprimés ou granulés dentaires, de gommes à mâcher ou de pâtes ou gels dentifrices, le véhicule étant choisi selon la forme désirée.

Les compositions à usage buccal peuvent donc contenir, outre le ou les composés de formule (I) utilisé selon l'invention, à titre de véhicule ou pour leur activité propre, des ingrédients habituellement utilisés dans les produits à usage buccal sous la forme correspondante.

Ces compositions sont préparées selon les procédés usuels correspondants aux véhicules choisis. Le véhicule physiologiquement acceptable peut être de différente nature selon la forme choisie pour la composition : solution aqueuse, solution hydroalcoolique épaissie ou non, gomme, excipient pâteux ou solide...

Dans ces compositions, les composés de formule (I) utilisés selon l'invention sont présents seuls ou en mélange, dans des quantités efficaces, c'est-à-dire à des concentrations pondérales comprises entre 0,0005 et 1%, et de préférence entre 0,005 et 0,1%.

Selon une réalisation préférée, les composés de formule (I) sont utilisés avec des agents antibactériens choisis de préférence parmi les agents actifs contre les germes gram négatifs dont certains sont des huiles essentielles. On peut citer à ce titre la chlorhexidine, l'alexidine, l'octinidine, l'hexétidine, l'hexamidine et leurs sels, le phénoxyéthanol, l'alcool phénéthylique, le triclosan, les chlorures de N-cétylpyridinium, de benzalkonium ou de benzéthonium.

Ces agents antibactériens peuvent représenter jusqu'à 10% en poids de la composition et sont présents de préférence à des concentrations comprises entre 0,001 et 2% en poids.

Selon les formes désirées, elles peuvent contenir en particulier au moins un agent de polissage dans des proportions allant jusqu'à 95%. Les agents de polissages sont des abrasifs qui peuvent être d'origine minérale ou organique. Leur nature peut différer selon le véhicule utilisé pour la forme choisie, comme cela apparaîtra ci-dessous.

De plus, dans certaines formes, les compositions peuvent contenir un ou plusieurs agent(s) tensio-actif(s). Les agents tensio-actifs utilisables peuvent être de nature anionique, amphotère, zwitterionique, cationique ou non-ionique, et on utilise de préférence des tensio-actifs anioniques ou non-ioniques.

Parmi les tensio-actifs anioniques, on peut citer les alkylsulfates et alkyléthersulfates tels que le laurylsulfate de sodium, le lauryléthersulfate de sodium, des N-méthyl (alkyl supérieur) taurates tel que le N-méthyl cocoyltaurate de sodium, des iséthionates tel que le cocoyliséthionate de sodium, des alkylarylsulfonates tel que le dodécylbenzène sulfonate de sodium, des alkylsulfoacétates supérieurs, des esters d'acides gras supérieurs de 1,2-dihydroxypropane sulfonates, des acylamides aliphatiques supérieurs (C₁₂-C₁₆) tels que par exemple la N-lauroylsarcosine et les sels de sodium, de potassium et d'éthanolamine de N-lauroyl, N-myristoyl, N-palmitoyl sarcosine.

Parmi les tensio-actifs non-ioniques, on peut citer les alkylphénols polyoxyéthylénés, les alcools polyoxyéthylénés, les esters polyoxyéthylénés d'acides gras, les alkylamines polyoxyéthylénés, les alkylamides polyoxyéthylénés, les esters de glycols ou de glycérol, les esters polyglycérolés, les esters de tétritol, de pentritol, d'hexitol ou d'anhydrohexitol, les esters de sucres, les esters de polyol polyoxyalkylénés, et les copolymères à motifs polyalkylène-oxyde, tels qu'en particulier les produits vendus sous les dénominations "PLURONIC" par la Société BASF et sous les dénominations "TERGITOL" par la Société UNION CARBIDE.

On peut utiliser en particulier les tensio-actifs non-ioniques de type poly(hydroxypropyléther) choisis parmi les composés de formules (II) à (IV) ci-après : 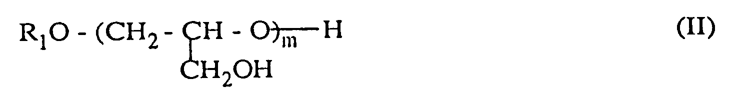
où R₁ désigne un radical ou un mélange de radicaux alkyles contenant 10 à 14 atomes de carbone et m est un nombre entier ou décimal de 2 à 10 et de préférence de 3 à 6, 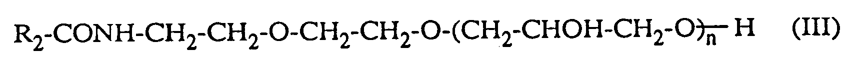
où R₂ désigne un radical ou un mélange de radicaux alkyles et/ou alcényles ayant de 11 à 17 atomes de carbone et n désigne un nombre entier ou décimal de 1 à 5 et de préférence de 1,5 à 4, 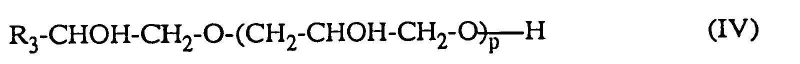
où R₃ désigne un radical aliphatique, cycloaliphatique, arylaliphatique, ayant de préférence 7 à 21 atomes de carbone, et leurs mélanges, les chaînes aliphatiques désignant en particulier des chaînes alkyle pouvant comporter 1 à 6 groupements éther, thioéther et/ou hydroxyméthylène et p est compris entre 1 et 10 inclus.

Les composés de formule (II) peuvent être préparés selon le procédé décrit dans le brevet FR 1.477.048, ceux de formule (III) peuvent l'être selon le procédé décrit dans le brevet FR 2.328.763 et ceux de formule (IV) peuvent être préparés par condensation en catalyse alcaline, de 2 à 10 et de préférence de 2,5 à 6 moles de glycidol sur un alpha-diol ou un mélange d'alpha-diols en C₁₀-C₁₄ à la température de 120-180°C et de préférence de 140 à 160°C, le glycidol étant ajouté lentement selon le procédé de préparation décrit dans le brevet FR 2.091.516.

De plus, on peut utiliser les tensio-actifs non-ioniques de type poly(hydroxypropyléther) préparés par condensation, en catalyse acide, de 2 à 10 et de préférence de 2,5 à 6 moles de glycidol par mole d'alcool ou d'alpha-diol contenant 10 à 14 atomes de carbone à une température de 50 à 120°C, le glycidol étant ajouté lentement à l'alcool ou à l'alpha-diol, le procédé de préparation de ces composés étant décrit dans le brevet FR 2.169.787, ou encore les composés préparés par poly-addition de monochlorhydrine du glycérol sur un composé organique poly(hydroxylé) en présence d'une base forte, avec élimination au fur et à mesure de l'eau par distillation, décrits en particulier dans le brevet français FR 2.574.786.

On peut également utiliser des tensio-actifs cationiques sous réserve qu'ils n'altèrent pas la stabilité des compositions.

Comme tensio-actif amphotère ou zwitterionique, on peut citer les dérivés d'amine tels que les produits vendus sous la dénomination "MIRANOL", décrits dans les brevets US-A 2,528,378 et 2,781,354 et classés dans le dictionnaire CTFA, 3ème édition, 1982, sous la dénomination d'Amphocarboxyglycinates et Amphocarboxypropionates.

De façon générale, les tensio-actifs peuvent être présents dans une gamme pondérale allant jusqu'à 20% par rapport à la composition et particulièrement dans la gamme de 0,5 à 5%.

En outre, les compositions comportant le ou les composés utilisés selon l'invention peuvent contenir d'autres agents actifs connus pour détruire la mauvaise haleine, tels que par exemple les cyclodextrines ou des composés du zinc qui sont des sels minéraux ou organiques tels que par exemple les halogénures de zinc, l'acétate de zinc, le citrate de zinc ou le fluorure de zinc.

Par ailleurs, elles peuvent contenir d'autres agents, usuels dans les compositions buccales, tels que les agents de cohésion, les agents moussants, les agents épaississants, les agents antibiotiques, les agents édulcorants, humectants ou rafraîchissants, les agents conservateurs, les agents aromatisants ou de sapidité, les agents peptisants, les agents plastifiants, les agents antibactériens, les agents anticaries, les agents antitartre, les cicatrisants, les vasomoteurs et les agents antisaignement. Ces différents agents sont contenus dans la composition, notamment selon la forme qu'elle prend.

Ainsi, lorsque la composition à usage buccal est un spray, le véhicule peut être une solution hydro-alcoolique et la composition peut contenir en outre des arômes, des agents peptisants, des agents édulcorants, humectants ou rafraîchissants.

De même, à titre d'exemple, lorsque la composition est sous forme de bains de bouche, le véhicule est essentiellement constitué par une solution aqueuse d'un agent moussant, éventuellement avec un agent épaississant, et peut comporter en outre des agents bactéricides, des édulcorants et des substances aromatisantes.

De plus, lorsque les compositions sont sous forme de gargarismes, elles peuvent renfermer un agent actif du type antibiotique.

Dans la forme de réalisation des compositions sous forme de poudre, comprimés et granulés, les compositions comprennent en général essentiellement un agent de polissage et un agent moussant.

Lorsque les compositions sont sous forme de gomme à mâcher, elles contiennent au moins une gomme masticable naturelle ou synthétique, et peuvent contenir des agents plastifiants, et des vitamines, des agents d'aromatisation ou de sapidité, des agents sucrants, des agents humectants, des bactéricides et des conservateurs et des colorants et éventuellement des agents de polissage.

Parmi les gommes ayant une élasticité suffisante, seules ou en mélanges, pour être masticables, on peut citer parmi les gommes naturelles le latex d'Hévéa, la gomme chicle, la gomme schulong et parmi les gommes de synthèse, l'acétate de polyvinyle et les élastomères de synthèse : caoutchouc silicone, caoutchouc butyl. Généralement, ces gommes contiennent 0,5 à 70% en poids de gomme masticable.

Les agents de polissage éventuellement utilisés usuellement dans les gommes à mâcher, peuvent être d'origine minérale ou organique. Ce sont par exemple les carbonate de calcium, de magnésium, de sodium, les phosphates et sulfates de calcium, l'alumine et l'alumine hydratée, les silices, les oxydes, hydroxydes, trisilicates et pyrophosphates de magnésium, ou des composés cellulosiques obtenus par broyage de graines de céréales.

Lorsque les compositions sont sous forme de pâte dentifrice, elles peuvent contenir un matériau de polissage abrasif minéral. Celui-ci est constitué par un ou plusieurs composés, en grande partie insolubles dans l'eau. On peut citer par exemple les métaphosphates de sodium ou de potassium, le phosphate de calcium dihydraté, le phosphate dicalcique, le phosphate tricalcique, le pyrophosphate de calcium, l'alumine, les alumines hydratées et en particulier trihydratées, les silices, les silicates d'aluminium ou de zirconium, la bentonite ainsi que l'orthophosphate de magnésium ou le phosphate trimagnésien.

Lorsque la composition est sous forme de gel transparent, à titre d'agents de polissage, on peut utiliser un agent de polissage à base de silice colloïdale ou d'aluminosilicates de métaux alcalins ou alcalino-terreux, et de préférence de sodium ou de calcium.

Dans les pâtes dentifrices, on peut également introduire des agents de cohésion. Ceux-ci peuvent être choisis parmi les gommes naturelles comme la gomme adragante, les gommes de xanthane, les gommes de guar, les gommes de caroube ou de carraghénanes ou comme des épaississants synthétiques, en général dérivés de cellulose comme le sel de sodium de la carboxyméthylcellulose, la méthylcellulose ou les hydroxyalkylcelluloses.

Lorsqu'ils sont présents, ces agents de cohésion sont généralement incorporés dans des proportions pouvant aller jusqu'à 10% en poids, et de préférence de 0,5 à 3% en poids.

Dans les compositions à usage buccal comportant un ou des composés utilisés selon de l'invention, on peut utiliser en outre un agent édulcorant. Parmi les agents édulcorants utilisables, on peut citer le saccharose, le lactose, le fructose, le xylitol, le cyclamate de sodium, le maltose ou le saccharinate de sodium, les mélanges d'α-glucosyl/steviolglucoside, le D-Mannitol, l'Aspartame, l'Acesulfam K et leurs mélanges.

Ces agents édulcorants sont alors présents dans des concentrations allant jusqu'à 2%.

Comme agents humectants, on peut citer le sorbitol, le glycérol ou le xylitol, présents à ce titre dans des concentrations pouvant atteindre 50%.

En outre, les agents rafraîchissants, comme le menthol ou l'éthylmaltol peuvent être incorporés.

Dans les compositions comportant le ou les composés utilisés selon l'invention, il est également possible d'utiliser des agents conservateurs pour assurer une bonne pureté bactériologique des formulations. Ceux-ci peuvent être choisis parmi le parahydroxybenzoate de méthyle, le parahydroxybenzoate de propyle, entre autres agents conservateurs usuels. Les concentrations peuvent alors aller jusqu'à 0,5% en poids.

Comme substance aromatisante pour les compositions comportant le ou les composés utilisés selon l'invention, on peut citer les essences de menthe, d'anis, d'eucalyptus, de cannelle, de girofle, de sauge, de réglisse, ou de fruits comme le citron, l'orange, la mandarine ou la fraise; on peut éventuellement utiliser à ce titre le salicylate de méthyle. Les substances aromatisantes, quand elles sont utilisées, peuvent l'être jusqu'à 5% en poids de la composition.

Enfin, il est possible d'incorporer dans les compositions à usage buccal comportant le ou les composés utilisés selon l'invention, des agents anticaries comme le monofluorophosphate de sodium, les fluorures de sodium ou d'étain, les amines fluorées, les fluorures de polymère cationique tels que ceux qui sont décrits dans le brevet français 2.647.012.

Enfin, les compositions peuvent contenir des agents actifs tels que des agents antitartre, des agents cicatrisants, des agents vasomoteurs ou des agents antisaignement.

L'invention concerne également un procédé d'hygiène buccale consistant à appliquer dans la cavité buccale un agent actif contre l'halitose ou la cacosmie, contenu dans une composition à usage buccal telle que décrite ci-dessus.

L'application peut être réalisée par gargarisme, bain de bouche, dispersion, projection, mastication ou brossage.

Les exemples présentés ci-après ne le sont qu'à titre indicatif, ils illustrent l'invention. Dans la description ci-dessus et les exemples les dénominations "PLURONIC", "TERGITOL", "MIRANOL", "G-SWEET", "TIXOSIL" et "BLANOSE" sont des marques déposées.

### EXEMPLE 1

Pour préparer un spray buccal, on mélange les composants suivants :

| | |
|---|---|
| - Alcool éthylique à 95° | 48 g |
| - Glycérol | 12 g |
| - Mélange d'α-glucosyl Steviolglucoside vendu sous la dénomination "G-SWEET" par la Société MAZUKEN | 0,02 g |
| - 2-crotonyl 1,1,3-triméthylcyclohexadiène-2,4 | 0,01 g |
| - Arôme | 1 g |
| - Eau | qsp 100 g |

### EXEMPLE 2

Pour préparer un spray buccal, on mélange les composants suivants :

| | |
|---|---|
| - Alcool éthylique à 95° | 45 g |
| - Glycérol | 20 g |
| - Menthol | 0,05 g |
| - 2-crotonyl 1,1,3-triméthylcyclohexadiène-2,4 | 0,005 g |
| - Peptisant | 1 g |
| - Arôme | 1 g |
| - Eau | qsp 100 g |

### EXEMPLE 3

Pour préparer une gomme à mâcher, on mélange les composants suivants :

| | |
|---|---|
| - Base gomme à mâcher | 20 g |
| - Agent édulcorant | 64 g |
| - Sirop de glucose | 15 g |
| - 2-crotonyl 1,1,3-triméthylcyclohexadiène-2,4 | 0,01 g |
| - Arôme qs | |

### EXEMPLE 4

Pour préparer un bain de bouche, on mélange les composants suivants :

| | |
|---|---|
| - Alcool éthylique | 10 g |
| - Saccharinate de sodium | 0,05 g |
| - Glycérol | 8 g |
| - Copolymère oxyde d'éthylène/oxyde de propylène, vendu sous la dénomination "PLURONIC F 108" par la Société BASF | 1 g |
| - 2-crotonyl 1,1,3-triméthylcyclohexadiène-2,4 | 0,015 g |
| - Peptisant | 1,5 g |
| - Arôme | 0,2 g |
| - Eau | qsp 200 g |

### EXEMPLE 5

Pour préparer une pâte dentifrice, on mélange les composants suivants :

| | |
|---|---|
| - Silice naturelle de Neubourg | 3,5 g |
| - Silices précipitées | 14 g |
| - Carraghénate de sodium | 1,2 g |
| - Dioxyde de titane | 1,5 g |
| - Fluorure de sodium | 0,33 g |
| - Laurylsulfate de sodium | 1,5 g |
| - Solution aqueuse de sorbitol à 70% | 17,5 g MA |
| - 2-crotonyl 1,1,3-triméthylcyclohexadiène-2,4 | 0,02 g |
| - Arôme | 1 g |
| - Eau | qsp 100 g |

### EXEMPLE 6

Pour préparer un gel dentifrice, on mélange les composants suivants :

| | |
|---|---|
| - Silice précipitée abrasive vendue sous la dénomination "TIXOSIL 73" par la Société RHONE POULENC | 13 g |
| - Silice précipitée gonflante vendue sous la dénomination "TIXOSIL 333" par la Société RHONE POULENC | 7 g |
| - Sorbitol en solution aqueuse à 70% de MA | 45,9 g MA |
| - Carboxyméthylcellulose de sodium vendue sous la dénomination " BLANOSE 12 M 31 X P" par la Société HERCULES | 0,8 g |
| - Monofluorophosphate de sodium | 0,76 g |
| - Laurylsulfate de sodium | 1,25 g |
| - 2-crotonyl 1,1,3-triméthyl cyclohexadiène-2,4 | 0,01 g |
| - Saccharinate de sodium | 0,2 g |
| - Conservateur, arôme, colorant qs | |
| - Eau | qsp 100 g |

## Revendications

1. Utilisation d'un composé de formule (I) : 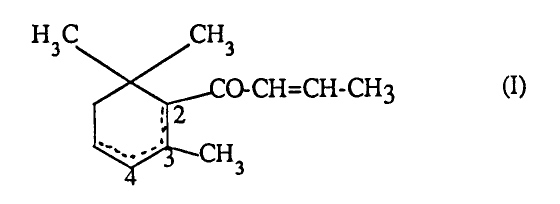 où les pointillés représentent une double en position 2 ou 3 ou deux doubles liaisons en positions 2 et 4, en vue d'inhiber ou détruire la mauvaise haleine au moyen d'une composition à usage buccal comportant au moins un desdits composés et un véhicule physiologiquement acceptable.

2. Utilisation selon la revendication 1, caractérisée en ce que le composé de formule (I) est le 2-crotonyl 1,1,3-triméthylcyclohexadiène-2,4.

3. Utilisation selon la revendication 1 ou 2, caratérisée en ce que la composition comporte 0,0005 à 1% en poids de composé de formule (I) par rapport à son poids total.

4. Utilisation selon l'une des revendications 1 à 3, caractérisée en ce que la composition comporte 0,005 à 0,1% en poids de composé de formule (I) par rapport à son poids total.

5. Utilisation selon l'une des revendications 1 à 4, caractérisée en ce que la composition comporte en outre jusqu'à 10% en poids d'un agent antibactérien par rapport à son poids total.

6. Utilisation selon l'une des revendications 1 à 5, caractérisée en ce que la composition est sous forme de spray, de mousse, de bain de bouche, de gargarisme, de poudre, de comprimé, de granulé dentaire, de gomme à mâcher, de pâte ou gel dentifrice.

7. Utilisation selon l'une des revendications 1 à 5, caractérisée en ce que la composition comporte en outre un agent de polissage présent dans des proportions allant jusqu'à 95% en poids par rapport au poids total de la composition.

8. Utilisation selon l'une des revendications 1 à 7, caractérisée en ce que la composition comporte en outre jusqu'à 20% en poids par rapport au poids total de la composition de tensio-actifs de nature anionique, non-ionique, amphotère, zwittérionique, cationique ou leurs mélanges.

9. Utilisation selon l'une des revendications 1 à 8, caractérisée en ce que la composition comporte en outre un autre agent actif inhibiteur de la mauvaise haleine, comme les cyclodextrines ou les composés du zinc.

10. Utilisation selon l'une revendications 1 à 9, caractérisée en ce que la composition comporte en outre au moins un agent de cohésion, un agent moussant, un agent épaississant, un agent édulcorant, un agent humectant, un agent rafraîchissant, une gomme masticable, un agent plastifiant, un agent conservateur, un agent aromatisant, un agent de sapidité, un antibiotique, un agent anticaries, un agent antitartre, un cicatrisant, un vasomoteur, un agent antisaignement ou un colorant.

11. Procédé d'hygiène buccale pour détruire la mauvaise haleine, caractérisé en ce qu'il consiste essentiellement à appliquer dans la cavité buccale une composition à usage buccal comprenant au moins un composé de formule (I) : 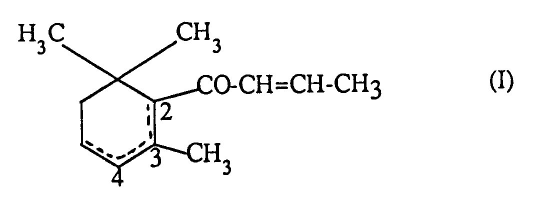 où les pointillés représentent une double liaison en position 2 ou 3 ou deux doubles liaisons en positions 2 et 4,
et un véhicule physiologiquement acceptable.

## Claims

1. Use of a compound of formula (I): 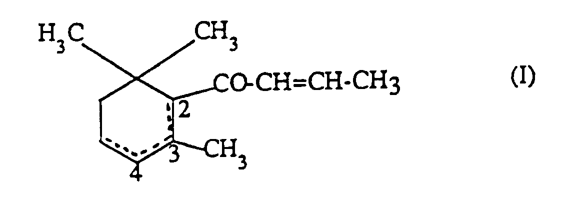 where the dotted lines represent a double bond at position 2 or 3 or two double bonds at positions 2 and 4, for the purpose of inhibiting or eradicating bad breath by means of a composition for buccal use containing at least one of the said compounds and a physiologically acceptable vehicle.

2. Use according to Claim 1, characterized in that the compound of formula (I) is 2-crotonyl-1,1,3-trimethyl-2,4-cyclohexadiene.

3. Use according to Claim 1 or 2, characterized in that the composition contains 0.0005 to 1% by weight of compound of formula (I) relative to its total weight.

4. Use according to one of Claims 1 to 3, characterized in that the composition contains 0.005 to 0.1% by weight of compound of formula (I) relative to its total weight.

5. Use according to one of Claims 1 to 4, characterized in that the composition contains, in addition, up to 10% by weight of an antibacterial agent relative to its total weight.

6. Use according to one of Claims 1 to 5, characterized in that the composition is in the form of a spray, foam, mouthwash, gargle, powder, tablet, dental granule, chewing gum, toothpaste or tooth gel.

7. Use according to one of Claims 1 to 5, characterized in that the composition contains, in addition, a polishing agent present in proportions ranging up to 95% by weight relative to the total weight of the composition.

8. Use according to one of Claims 1 to 7, characterized in that the composition contains, in addition, up to 20% by weight, relative to the total weight of the composition, of surfactants which are anionic, nonionic, amphoteric, zwitterionic or cationic in nature, or mixtures thereof.

9. Use according to one of Claims 1 to 8, characterized in that the composition contains, in addition, another active agent which inhibits bad breath, such as cyclodextrins or zinc compounds.

10. Use according to one of Claims 1 to 9, characterized in that the composition contains, in addition, at least one binding agent, foaming agent, thickening agent, sweetening agent, humectant agent, freshening agent, chewable gum, plasticizing agent, preservative, flavouring agent, flavour modifier, antibiotic, anticaries agent, antitartar agent, cicatrizing agent, vasomotor agent, haemostatic agent or colorant.

11. Buccal hygiene process for eradicating bad breath, characterized in that it consists essentially in applying in the buccal cavity a composition for buccal use comprising at least one compound of formula (I): 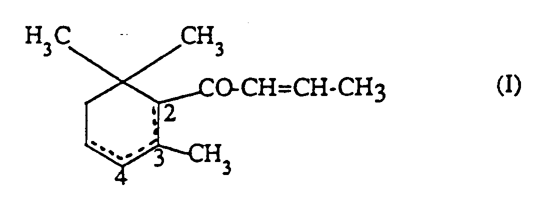 where the dotted lines represent a double bond at position 2 or 3 or two double bonds at positions 2 and 4, and a physiologically acceptable vehicle.

## Patentansprüche

1. Verwendung einer Verbindung der Formel (I): 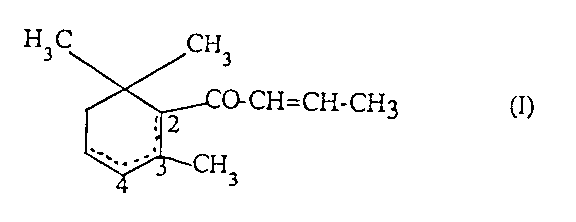 worin die Pünktchen eine Doppelbindung in Position 2 oder 3 oder zwei Doppelbindungen in Positionen 2 und 4 darstellen,
zur Inhibierung oder Zerstörung von Mundgeruch und schlechtem Atem mittels einer zur Anwendung im Mundbereich vorgesehenen Zusammensetzung, welche mindestens eine der genannten Verbindungen und ein physiologisch zulässiges Trägermittel enthält.

2. Verwendung gemäß Anspruch 1,
dadurch **gekennzeichnet**, daß
die Verbindung der Formel (I) 2-Crotonyl-1,1,3-trimethylcyclohexa-2,4-dien ist.

3. Verwendung gemäß Anspruch 1 oder 2,
dadurch **gekennzeichnet**, daß
die Zusammensetzung 0,0005 bis 1 Gew.% Verbindung der Formel (I) enthält, bezogen auf ihr Gesamtgewicht.

4. Verwendung gemäß einem der Ansprüche 1 bis 3,
dadurch **gekennzeichnet**, daß
die Zusammensetzung 0,005 bis 1 Gew.% Verbindung der Formel (I) entählt, bezogen auf ihr Gesamtgewicht.

5. Verwendung gemäß einem der Ansprüche 1 bis 4,
dadurch **gekennzeichnet**, daß
die Zusammensetzung ausserdem bis zu 10 Gew.% eines antibakteriellen Mittels enthält, bezogen auf ihr Gesamtgewicht.

6. Verwendung gemäß einem der Ansprüche 1 bis 5,
dadurch **gekennzeichnet**, daß
sie in Form eines Spray, Schaums, Mundbads, Mittel zum Gurgeln, Pulvers, einer Tablette oder eines Granulats für die Zähne, eines Kaugummi, einer Zahnpaste oder eines Zahnputzmittelgels vorliegt.

7. Verwendung gemäß einem der Ansprüche 1 bis 5,
dadurch **gekennzeichnet**, daß
sie ausserdem ein Poliermittel enthält, das in Mengenanteilen bis zu 95 Gew.% vorhanden ist, bezogen auf das Gesamtgewicht der Zusammensetzung.

8. Verwendung gemäß einem der Ansprüche 1 bis 7,
dadurch **gekennzeichnet**, daß
die Zusammensetzung ausserdem bis zu 20 Gew.%, bezogen auf das Gesamtgewicht der Zusammensetzung, oberflächenaktive Mittel anionischer, nicht-ionischer, amphoterer, zwitterionischer oder kationischer Natur oder deren Mischungen enthält.

9. Verwendung gemäß einem der Ansprüche 1 bis 8,
dadurch **gekennzeichnet**, daß
die Zusammensetzung ausserdem ein weiteres Inhibitor-Wirkstoffmittel gegen Mundgeruch und schlechten Atem, wie Cyclodextrine oder Zinkverbindungen, enthält.

10. Verwendung gemäß einem der Ansprüche 1 bis 9,
dadurch **gekennzeichnet**, daß
die Zusammensetzung ausserdem mindestens ein Kohäsions-, Schäumungs-, Verdickungs-, Süßungs-, Feuchtigkeits-, Erfrischungs-, Kaugummi-, Plastifizier-, Konservierungs-, Aromatisierungs-, Geschmacksverbesserungs-, Antibiotikum-, Antikaries-, Antizahnstein-, Wund-, Vasomotorik-, Antiblutungs- oder Färbemittel enthält.

11. Verfahren zur Hygienebehandlung des Mundbereichs zur Zerstörung von Mundgeruch und schlechtem Atem,
dadurch **gekennzeichnet**, daß
es im wesentlichen darauf beruht, daß man in die Mundhöhle eine zur Anwendung im Mundbereich vorgesehene Zusammensetzung einbringt und dort einwirken läßt, welche mindestens eine Verbindung der Formel (I): 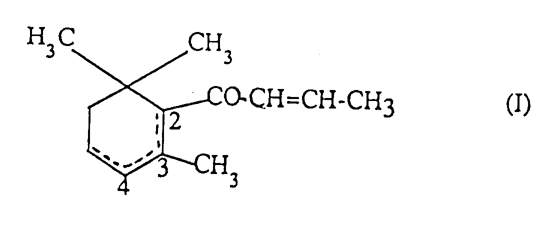 worin die Pünktchen eine Doppelbindung in Position 2 oder 3 oder zwei Doppelbindungen in Positionen 2 und 4 darstellen, sowie ein phyiologisch geeignetes Trägermittel enthält.
